# EUROPEAN PATENT SPECIFICATION

(11) **EP 3 257 516 B1**
(45) Date of publication and mention of the grant of the patent: **31.07.2019**
(21) Application number: 17176138.0
(22) Date of filing: 14.06.2017
(51) Int. Cl.: A61K 36/09, A61K 31/728, A61K 33/14, A61K 9/12, A61P 11/00

(54) **PHARMACEUTICAL COMPOSITION COMPRISING CETRARIA ISLANDICA ACH., SODIUM HYALURONATE AND A SALINE SOLUTION FOR TREATING AILMENTS OF THE RESPIRATORY SYSTEM**
PHARMAZEUTISCHE ZUSAMMENSETZUNG ENTHALTEND CETRARIA ISLANDICA ACH., NATRIUMHYALURONAT UND EINE SALINE LÖSUNG ZUR BEHANDLUNG VON ERKRANKUNGEN DES RESPIRATORISCHEN SYSTEMS
COMPOSITION PHARMACEUTIQUE COMPRENANT CETRARIA ISLANDICA ACH., DE HYALURONATE DE SODIUM ET UNE SOLUTION SALINE POUR TRAITER DES MALADIES DU SYSTÈME RESPIRATOIRE

(30) Priority: 15.06.2016 IT UA20164387
(43) Date of publication of application: 20.12.2017
(73) Proprietor: Neilos S.r.l., 80063 Piano di Sorrento (NA) (IT)
(72) Inventor: DI MAIO, Umberto, 80063 Piano di Sorrento (NA) (IT); BAGNULO, Antonino,, 80063 Piano di Sorrento (NA) (IT)
(74) Representative: Hoffmann Eitle

(56) References cited:
- EP-A1- 2 664 330
- WO-A1-2015/082356
- DE-U1-202006 011 920
- DE-U1-202013 000 445
- Marie Heroutova ET AL: "Assessment report on Cetraria islandica (L.) Acharius s.l., thallus" In: "EMA/HMPC/36866/2014", 6 May 2014 (2014-05-06), XP055349638, * page 4 - page 10 * * page 14 - page 18 * * page 22; tables 5,6 *

## Description

The present invention relates to a pharmaceutical composition comprising a mixture that comprises or, alternatively, consists of *Cetraria islandica Ach.,* sodium hyaluronate and a saline solution with an osmolarity such as to ensure that the finished product has an osmolarity greater than or equal to 300 mOsm/l and, for use as a medicament, preferably for adolescents and subjects of paediatric age, in the treatment of respiratory tract disorders/pathologies selected from among rhinitis, sinusitis, asthma, pharyngitis (pharyngotonsillitis), epiglottitis, laryngitis, bronchiolitis, cystic fibrosis and bronchiectasis, preferably for adolescents and subjects of paediatric age. Finally, a preferred embodiment of the present invention relates to said composition for use in the treatment of respiratory tract pathologies associated with mucus hypersecretion, preferably for adolescents and subjects of paediatric age.

It is well known that the respiratory tract consists of various anatomical structures necessary for its correct functioning. It has the task, in fact, of exchanging gases, oxygen and carbon dioxide, between tissues and the outside environment, this function being fundamental for all of the body's cellular processes. Anatomically, two macro areas can be distinguished, the upper and lower airways.

The upper airways consist of the nose, pharynx and associated structures, whilst the lower airways consist of the larynx, trachea, bronchi and lungs, whose actual respiratory surface consists in the alveoli. This complex system of organs serves to prepare the air for entry into the lungs by filtering it from any particulate matter and heating and humidifying it. The presence of mucus and particular cell structures (such as the hairs of the nose) bring about the filtration of air and only particles of a few microns with an appropriate aerodynamic diameter are allowed to pass.

The entire respiratory tract consists of epithelial cells along the tracheobronchial tree which differ in type and function. Ciliated columnar cells characterise the airways from the trachea to the terminal bronchioles. Muciferous goblet cells (present in the bronchi) have the function of secreting mucus, useful for maintaining the correct moisture of the epithelium and trapping particulate matter.

The most common respiratory tract disorders/pathologies are described below.

Rhinitis: it is an inflammatory process affecting the mucosa of the nasal cavities and may be distinguished between acute and chronic. Acute rhinitis is generally sustained by viruses, including Rhinovirus, influenza and parainfluenza virus, RSV, Coxsackie virus, and adenovirus. It is possible for there to be bacterial superinfections, which lead to complications such as otitis and sinusitis. The common cold caused by Rhinovirus gives rise to acute symptoms in the first days, with an excess of mucus secretions that are fluid and transparent, and become purulent and foul smelling in the event of bacterial superimposition.

The chronic form is generally secondary to sinusitis, nasal septum deviations and hypertrophic adenoids.

Allergic rhinitis is of the acute type and is due to exposure of a subject to substances that provoke an IgE-mediated reaction, characterised by an excessive production of fluids, intranasal itching, sneezing and obstruction. Recent studies have highlighted a correlation in the manifestation of allergic rhinitis and asthma, which should be considered as two manifestations of the entire respiratory tract, rather than attributing one to the upper tract and the other to the lower tract of the respiratory tree.

Sinusitis: it is an inflammation of the mucosa which coats the paranasal sinuses, bone cavities that are situated in the facial skeleton and are in communication with the nasal fossae. When sinusitis involves the nasal cavity one speaks of rhinosinusitis. Ciliary abnormalities or immobility determine an inhibition of drainage resulting in sinusitis. Factors predisposing to this pathology are an immunocompromised state, nasal septum deviation, nasal polyps, tumours, traumas and fractures, cocaine abuse and the presence of foreign bodies. An acute viral form can be susceptible to bacterial superinfection. Fungal infections are also possible. The signs and symptoms of acute rhinosinusitis consist in: *mucopurulent* discharges from the nose, nasal obstruction, congestion, facial pain, hyposmia, anosmia and fever. Chronic forms have a slower onset, longer duration and greater frequency.

Pharyngitis (pharyngotonsillitis): it is an inflammatory process of the pharynx, hypopharynx, uvula and tonsils, which is generally transmitted by direct contact with respiratory secretions. It is more frequent in paediatric age (5-15 years) and although it is often self-limiting, the swelling of the parts involved can cause a reduced patency of the airways or in any case preclude the ingestion of adequate amounts of liquids, with consequent dehydration. The infection can be sustained by viruses (such as Epstein-Barr) and bacteria (such as Group A beta-haemolytic *Streptococcus pyogenes, C. diphtheriae*).

Epiglottitis: it is an inflammation of the epiglottis, caused by a viral or bacterial infection, which determines a swelling of the organ with a possible obstruction of the airways. It is caused mainly by *H. influenzae* type b, but also by streptococci, staphylococci or a thermal trauma. It manifests itself with ear pain (in adults) and dysphonia, whilst fever is absent in up to 50% of cases and can develop at a late stage.

Laryngitis: it is an inflammation that manifests itself with aphonia and hoarseness, caused principally by viruses, but also by bacteria (including streptococci and C. *diphtheriae*) in up to 10% of cases. Non-infectious causes can be tumours, a thermal or caustic trauma or GERD. Asthma: it is a common inflammatory pathology, often also paediatric, whose etiology is influenced by genetic and environmental factors. Characterised by hyperreactivity of the airways, which is manifested as an excessive obstruction in response to bronchoconstrictors and allergens, overproduction of mucus (due to the secretion of mediators by mast cells and neutrophils) and infiltration of eosinophils, it affects over 300 million people in the world. Bronchiolitis: a frequent disease in paediatric age, it is characterised by an extensive inflammation of the airways accompanied by an intense production of mucus and necrosis of epithelial cells. It is primarily caused by a viral infection, RSV in particular, but also adenovirus, influenza and parainfluenza viruses and rhinovirus, whereas the most frequently involved bacteria are of the genus Clamidia. In paediatric age the principal clinical manifestations are tachypnea, breathlessness, or crackles on auscultation, which generally follow an infection of the upper respiratory tract.

Cystic fibrosis: it is a disease caused by a mutation of the gene that codes for the protein CFTR, an anion channel expressed in epithelial cells throughout the body. Although it functions above all as a chloride ion channel, it is also capable of regulating the function of other membrane proteins, such the epithelial sodium channel (ENaC), whose activity is inhibited. The dysfunction of the CFTR channel in the lungs determines an excessive absorption of sodium and a reduced active secretion of chlorine, with a consequent reduction in the liquid layer on the surface of the mucosa. This leads to an anomalous mucus-ciliary clearance, with a retention of viscous mucus, which favours infections and inflammation and thus lung damage.

Bronchiectasis: it is a pathology characterised by an irreversible dilation of a portion of the bronchial tree in the lungs. Bronchial dilation can be the result of a structural defect of the wall, exposure to an abnormal pressure, or else damage to the cartilage or elastic tissue as a result of an inflammation.

It affects the bronchi and bronchioles, where a vicious circle of infection and inflammation can arise, also with the release of mediators. Common symptoms are coughing up mucus and chest pain. The mucus contains an increased amount of elastase, TNF a, IL-8 and prostanoids. It can manifest itself as a local or diffuse obstructive process involving part of both lobes, also accompanied by sinusitis or asthma.

On the market there are many mucolytic products containing or based on, for example, the active ingredient N-acetylcysteine, which acts by breaking disulphide bridges. However, the effectiveness of mucolytic products in general, and N-acetylcysteine in particular, is not always decisive and often, as in the case of the active ingredient N-acetylcysteine, the use thereof is contraindicated in children under two years of age.

Erdosteine is a mucolytic agent also capable of reducing the adhesiveness of bacteria and guaifenesin can reduce the surface tension of mucus, with an expectorant effect. However, the use of these active ingredients as well has not always given clear, definitive results with an appreciable effectiveness.

Other products present on the market are 0.9% NaCl solution in vials, hypertonic solutions, solutions with hyaluronic acid and combinations of these. However, saline solution on its own, for example, can only exert a cleansing effect on the mucosa or may serve to facilitate the delivery of other drugs.

Document DE 20 2006 011920 U1 discloses a composition for treating rhinitis comprising (a) sodium chloride, (b) Icelandic moss (Lichen islandicus), also known as Cetraria Islandica and, optionally, (c) hyaluronic acid or its salts. In addition, D1 discloses in Example 1 a composition comprising (b) Lichen islandicus and (a) sodium chloride that guarantees a osmolarity equal to 300 mOsm/L. However, the composition of Example 1 of such document does not contain hyaluronic acid or its salts.

Document DE 20 2013 000445 U1 discloses a composition for treatment of hoarseness or inflammatory diseases of oropharynx (sore throat) comprising (a) at least one polysaccharide, such as Icelandic moss (Lichen islandicus), also known as Cetraria Islandica, and (b) at least one glycosaminoglycan, preferably hyaluronic acid or its salts, more preferably sodium hyaluronic. However, such document is silent about a composition comprising, besides Cetraria Islandica and hyaluronic acid or its sodium salt, also a saline solution that guarantees a osmolarity greater than or equal to 300 mOsm/L.

Thus there is still a greatly felt need among practitioners in the field to be able to have new compositions for treating respiratory tract disorders/pathologies which are efficacious, easy to use, free of side effects and effective for all categories of patients, including adolescents and subjects of paediatric age.

The Applicant, after a long and extensive research and development activity, has provided an answer to the above-mentioned needs by developing a new composition for the treatment of upper and lower respiratory tract disorders/pathologies.

The present invention relates to the composition for use as claimed in the appended claims.

Preferred embodiments of the present invention are described below by way of example and hence do not limit the scope of the invention.

Unless specified otherwise, within the scope of the present invention the percentages and amounts of a component in a mixture are intended to refer to the weight of that component relative to the total weight of the mixture.

Within the scope of the present invention, "treatment" of diseases means a therapy aimed at restoring a subject's conditions of health, maintaining the existing conditions and/or preventing said conditions of health from worsening.

Within the scope of the present invention, "prevention" of pathologies means a therapy aimed at preventing the occurrence of such a pathology in a subject, also, but not only, as a complication or effect of a pathological condition or pre-existing disorder.

In the context of the present invention, composition(s) means any pharmaceutical form and, by way of example, pharmaceutical compositions in a spray or vials, or medical devices in a spray or vials, all indistinctly for oral, nasal or inhalatory use by spray or aerosol application.

The present invention relates to a liquid pharmaceutical composition for use which comprises a mixture and, optionally, pharmaceutically acceptable excipients, additives and ingredients, for the treatment of upper and lower respiratory tract disorders/pathologies.

By way of non-limiting example, use is made of the following excipients, which will be added to the mixture of the present invention:
- Sodium hydroxide;
- Citric acid;
- Sodium benzoate;
- Potassium sorbate;
- Sodium dehydroacetate;
- Polysorbate 20.

The present invention relates to a pharmaceutical composition comprising a mixture which comprises or, alternatively, consists of *Cetraria islandica Ach.,* sodium hyaluronate and a saline solution with an osmolarity such as to ensure that the finished product/final composition has an osmolarity greater than or equal to 300 mOsm/l and, optionally, pharmaceutically acceptable excipients, additives and ingredients; for use as a medicament in the treatment of respiratory tract disorders/pathologies selected from among rhinitis, sinusitis, asthma, pharyngitis (pharyngotonsillitis), epiglottitis, laryngitis, bronchiolitis, cystic fibrosis and bronchiectasis.

The composition for use of the present invention advantageously shows an efficacious therapeutic effect thanks to the synergistic action of the components/active ingredients contained therein.

*Cetraria islandica Ach.,* as a fluid, dry or glyceric extract, contained in the mixture, which is in turn contained in the composition for use of the present invention, is advantageously present in a concentration by weight comprised from 0.001% to 10% relative to the finished product/final composition, preferably in a concentration by weight comprised from 0.001% to 5%, and even more preferably in a concentration by weight comprised from 0.001 % to 2.5%.

Sodium hyaluronate is present together with the *Cetraria islandica Ach.* in an optional concentration by weight comprised from 0.01% to 5%, preferably in a concentration by weight comprised from 0.05% to 2.50%, or from 0.3% to 2.5%, and even more preferably in a concentration by weight comprised from 0.1% to 1% or from 0.5% to 1%, relative to the finished product/final composition.

The average molecular weight of the sodium hyaluronate, used together with the *Cetraria islandica Ach.,* is advantageously comprised from 100 kDa to 3000 kDa; preferably, the average molecular weight of the sodium hyaluronate is comprised from 200 kDa to 1500 kDa; even more preferably, the average molecular weight of the sodium hyaluronate is comprised from 300 kDa to 1000 kDa, for example from 500 kDa to 800 kDa.

In the context of the present invention, sodium hyaluronate is meant also to include hyaluronic acid and/or salts thereof, such as salts of alkali or alkaline earth metals, e.g. potassium, magnesium or calcium.

The composition for use of the present invention is in liquid form and contains, in addition to *Cetraria islandica Ach.* and sodium hyaluronate, a saline solution based on an osmotically active salt, such as, for example, sodium chloride. The saline solution contained in the composition for use of the present invention has an osmolarity such as to ensure that the finished product/final composition has an osmolarity greater than or equal to 300 mOsm/l, preferably comprised from 300 mOsm/l to 10000 mOsm/l or from 300 mOsm/l to 2000 mOsm/l; even more preferably it has an osmolarity comprised from 300 mOsm/l to 3000 mOsm/l or from 400 mOsm/l to 1000 mOsm/l, for example 360, 500, 600, 700, 800, 900, 1000, 1220, 1660, 2100, 2380 or 2900 mOsm/l.

The composition of the present invention is for use as a medicament and is advantageously indicated for the treatment of upper and lower respiratory tract disorders/pathologies, in all categories of patients, in particular for the treatment of adolescents and subjects of paediatric age.

The composition of the present invention is a composition for use as a medicament and can be validly employed for nasal use by spray or aerosol application in the treatment of upper and lower respiratory tract disorders/pathologies selected from among rhinitis, sinusitis, asthma, pharyngitis (pharyngotonsillitis), epiglottitis, laryngitis, bronchiolitis, cystic fibrosis and bronchiectasis.

The composition for use of the present invention is advantageously for oral, nasal or inhalatory use and is preferably administered by nebulization with a special device, such as a pneumatic or ultrasonic nebulizer, or by using a spray, or by direct nasal instillation.

Advantageously, the composition for use of the present invention makes it possible to obtain an effective (a) expectorant effect thanks to a i) reduction in the viscosity of mucus and/or ii) reduction in the adhesiveness of mucus and/or iii) increase in mucociliary clearance.

Furthermore, the composition for use of the present invention advantageously also shows an (b) antibacterial effect, and/or (c) anti-inflammatory effect, and/or (d) antioxidant effect.

Thanks to fumarprotocetraric acid, *Cetraria islandica* is capable of reducing the viscosity of mucus. Thanks to lichesterinic, protolichesterinic and rocellaric acids it can lead to a reduction in the surface tension of mucus and in this manner reduce the adhesiveness thereof to the respiratory mucosa. The sodium chloride solution has an osmotic action that enables water to be drawn in, with a consequent increase in the fluidity of mucus. Furthermore, it is capable of leading to a breakage of the ionic bonds of mucins. Finally, it has shown an ability to reduce the adhesion of DNA to mucins, thereby facilitating the action of proteolytic enzymes in the degradation of mucins as well as that of DNAase in the degradation of DNA, which leads to a reduction in the viscosity of mucus. These three actions lead to a reduction in the viscosity of mucus. Sodium hyaluronate is capable of increasing mucociliary clearance. The combination of the effects of reducing the viscosity of mucus, reducing the adhesiveness of mucus and increasing mucociliary clearance leads to an increase in expectoration.

The extract of *Cetraria islandica* exhibits antibacterial and antimycotic actions, whose mechanism has not yet been made clear. Sodium hyaluronate is capable of reducing the adhesiveness of bacteria to the respiratory mucosa. These two effects contribute to combating bacterial and fungal respiratory tract infections. *Cetraria islandica* has an antioxidant action, mainly associated with fumarprotocetraric acid, which can contribute to reducing oxidative stress and hence the inflammation associated with the pathologies that can affect the respiratory tract.

Finally, *Cetraria islandica* also exhibits an anti-inflammatory action thanks to lichenans, which increase the production of IL-10, which in turn inhibits the synthesis and release of pro-inflammatory cytokines. This can contribute to slowing the progression of pathologies that affect the respiratory tract and reducing the symptoms associated therewith.

The combination of the expectorant, antibacterial, anti-inflammatory and antioxidant effects of the active ingredients used in this formulation can lead to a therapeutic efficacy in the treatment of the respiratory tract pathologies associated with mucus hypersecretion, and a substantial improvement in respiratory activity.

The extracts of *Cetraria Islandica Ach.* in cetyilic alcohol that could be used in an embodiment of the composition for use of the present invention are in the form of a fluid, dry or glyceric extract. Extracts of *Cetraria islandica* (*Cetraria islandica* (L.) Ach., or *Iceland moss,* is a species of fruticose lichen that grows on soil, typical of mountainous areas) contain a high concentration of fumarprotocetraric acid, which can reach 11.5% in the dry extract. Fumarprotocetraric acid possesses an expectorant and antioxidant action, as well as an antibacterial and anti-inflammatory one. The expectorant activity was evaluated using the phenol red method. The oral administration of increasing doses of fumarprotocetraric acid led to an increase in the amount of mucus secreted, which was 5.03, 6.4 and 8.8 times higher than basal secretion at dosages of 25 mg/kg, 50 mg/kg and 100 mg/kg.

*Cetraria islandica* contains various secondary metabolites, each of which could have a physiological role. The main one is fumarprotocetraric acid, but also present are: lichesterinic acid, protolichesterinic acid, rocellaric acid and protocetraric acid. From a structural viewpoint, lichesterinic, protolichesterinic and rocellaric acids are surfactants; it is thus probable that they act by reducing the surface tension of mucus. In this manner, the viscosity of mucus and its capacity to adhere to epithelial cells would be reduced, thus favouring the elimination thereof.

In addition to the mechanisms of action involved in the expectorant activity, the extracts of *Cetraria* also exhibit an antibacterial, antimycotic and anti-inflammatory action, which can contribute to its therapeutic effectiveness in the treatment of respiratory pathologies.

Hyaluronic acid is a polymer consisting of D-glucuronic acid and D-N-acetylglucosamine, joined by β-1,4 and β-1,3 glycosidic bonds. It is a component of the normal secretions of the airways. It is secreted by the serous cells of the submucosal gland of the airways and the cells of the surface epithelium.

The molecular weight of the polymer determines its biological activity. In fact, low molecular weight hyaluronic acid (< 1000 kDa) is capable of modifying the beat frequency of the cilia present on the epithelial cells of the trachea.

The use of a hypertonic saline solution as an adjuvant in numerous respiratory tract problems is by now common. In numerous pathological situations, such as, for example, bronchiectasis, the presence of excess mucus represents one of the most important factors in keeping the infection and inflammation active, with consequent negative impacts on the quality of life as well as an increased morbidity and mortality. A hypertonic solution of sodium chloride is capable of destroying the ionic bonds present within mucus, which can reduce the viscosity thereof by reducing the cross-links.

In one embodiment (FR1), the present invention relates to a pharmaceutical composition comprising:
- a mixture which comprises or, alternatively, consists of *Cetraria islandica Ach.,* sodium hyaluronate and a saline solution with an osmolarity such as to ensure that said composition has an osmolarity greater than or equal to 300 mOsm/l and, optionally,
- pharmaceutically acceptable excipients, additives and ingredients;
for use as a medicament in the treatment of respiratory tract disorders/pathologies selected from among rhinitis, sinusitis, asthma, pharyngitis (pharyngotonsillitis), epiglottitis, laryngitis, bronchiolitis, cystic fibrosis and bronchiectasis.

In a preferred embodiment (FR2), in the composition for use according to FR1, *Cetraria islandica Ach.* is present as a fluid, dry or glyceric extract; preferably the *Cetraria islandica Ach.* is present in a concentration by weight comprised from 0.001% to 10%, preferably in a concentration by weight comprised from 0.1% to 5%, even more preferably in a concentration by weight comprised from 0.1% to 2.5%, relative to the total weight of the composition.

In a preferred embodiment (FR3), in the composition for use according to FR1 or FR2, the sodium hyaluronate is present in a concentration by weight comprised from 0.1% to 5%, preferably in a concentration by weight comprised from 0.3% to 2.5%, even more preferably in a concentration by weight comprised from 0.5% to 1%, relative to the total weight of the composition.

In a preferred embodiment (FR4), in the composition for use according to one of FR1-FR3, the average molecular weight of the sodium hyaluronate used together with *Cetraria islandica Ach.* is comprised from 100 kDa to 3000 kDa; preferably, the average molecular weight of the sodium hyaluronate is comprised from 200 kDa to 1500 kDa; even more preferably, the average molecular weight of the sodium hyaluronate is comprised from 300 kDa to 1000 kDa.

In a preferred embodiment (FR5), in the composition for use according to one of FR1-FR4, the saline solution is based on an osmotically active salt, preferably sodium chloride, in an amount such as to ensure that said composition has an osmolarity greater than or equal to 300 mOsm/l, preferably comprised from 300 mOsm/l to 2000 mOsm/l; even more preferably, it has an osmolarity comprised from 400 mOsm/l to 1000 mOsm/l.

In a preferred embodiment (FR6), in the composition for use according to one of FR1-FR5, said composition for use as a medicament is preferably for the treatment of adolescents and subjects of paediatric age.

In a preferred embodiment (FR8), in the composition for use according to one of FR1-FR6, said composition for use as a medicament is for the treatment of the respiratory tract pathologies associated with mucus hypersecretion, preferably in adolescents and subjects of paediatric age.

In a preferred embodiment (FR9), in the composition for use according to one of FR1-FR6 or FR8, said composition is for oral or nasal use by spray or aerosol application/administration.

In a preferred embodiment (FR10), in the composition for use according to one of FR1-FR6 or FR8-FR9, said composition is administered by nebulization with a special device, such as a pneumatic or ultrasonic nebulizer, or by using a spray, or by direct nasal instillation.

### MATERIALS AND METHODS

### 1. Expectorant activity

The expectorant activity can be evaluated using the phenol red method, a method amply described in the literature [1]-[6].

The administration of the formulation by inhalation can be carried out by intubation of the trachea of rats, following the method described by Lizio et al. [7]. For the study use was made of male Sprague-Dawley rats, which weigh from 250 to 300g. The administration of the formulation is carried out after a period of acclimatisation lasting about a week, during which they are housed in temperature controlled rooms (temperature of 23±2°C, humidity of 50±2%, light-dark cycles of 12 hours), with free access to water and food. In order to be able to intubate the trachea of the rats, a dose of atropine is administered (50 mg/kg, intramuscularly) under slight anaesthesia performed with carbon dioxide, as described by Koehler et al. [8]. The administration of atropine serves to reduce the sensitivity and motility of the larynx and the secretion of saliva, thereby facilitating the intubation. Ten minutes after the administration of atropine, a solution containing xylazine (8 mg/kg) and ketamine (80 mg/kg) is administered to induce anaesthesia. The rats are suspended by the upper incisors to a metal bar attached to a table inclined by 30° relative to the supine position. In order to be able to have the larynx, pharynx and trachea on the same plane, a metal cylinder is placed behind the neck of the animals. The mouth can be held in an open position using flat tweezers or a small forceps. The trachea can be visualised thanks to percutaneous illumination, obtained using a 12 V-high intensity lamp. The light of the lamp is conveyed by means of a fibre optic rod placed in contact with the skin in the pharyngoepiglottic region of the rat's neck, in order to make the vocal cords and lumen of the trachea visible. For the intubation, use is made of a 16 gauge needle, modified in such a way as to be 3 mm shorter than the polyethylene catheter and act as a "stylet". The distal part of the catheter is cut diagonally with an inclination of 30°. In order to be able to insert the catheter in the trachea, the following operation is carried out: the tip of the tube is positioned to the right, then introduced between the rat's vocal cords and finally rotated by 90° before being completely inserted into the trachea, in such a way as to have the tip inserted directly into the tracheal lumen. At this point, the needle can be removed and the catheter made to advance [7].

Thirty minutes after the administration of the formulation by inhalation, a 10 mg/mL solution of phenol red (phenylsulfonphthalein, an acid-base indicator) is administered intraperitoneally at a dose of 200 mg/mL. Thirty minutes after the administration of phenol red, the animals are anaesthetised; the anterosuperior part of the neck is shaven and the trachea is exposed. A tracheobronchial lavage is performed with 2 mL of 0.9% saline solution and 1 mL of fluid is recovered after the lavage. The lavage fluid is centrifuged at 1600 rpm for 10 minutes. At this point, the supernatant is collected and 0.5 mL of a solution of NaOH 0.01 N is added to it to permit the indicator to change colour. The concentration of phenol red is determined by means of a spectrophotometer at a wavelength of 535 nm and expressed in mg/mL [6].

An alternative procedure for carrying out the phenol red test has been described by Kee Jae Song et al. In this case, after the administration of the formulation, a 0.2 mL solution of 2.5% phenol red is injected. After 30 minutes the rats are sacrificed. The trachea is dissected and immersed in 0.9% saline solution. Subsequently, the trachea is lavaged and 0.1 mL of sodium hydroxide solution 1M is added to the saline solution. The concentration is measured by means of a spectrophotometer at a wavelength of 546 nm.

Alternatively, the expectorant activity of the various formulations containing *Cetraria islandica* can be evaluated in mice using the phenol red method, as amply described in the literature (Engler H & Szelenyi I, J Pharmacol Methods, 1984; 11:151-157*;* Coppi G & Gatti MT, Farmaco. 1989; 44:541-5*;* de Barros Alves GM et al., Pulm Pharmacol Ther. 2014; 27:139-43).

The mice (Charles River, 20 - 25 g males), after a period of acclimatisation lasting about a week, during which they are housed in temperature controlled rooms (temperature of 23±2 °C, humidity of 50±2%, light-dark cycles of 12 hours), with free access to water and food, are distributed into groups of 6-10 animals each and receive the formulations to be tested via aerosol (5 ml) (see table 2). A control group (positive control) receives guaifenesin as the reference drug (500 mg/kg per os). Some animals receive no treatment (naive animals). After 30 minutes have elapsed following the administration, each mouse receives, intraperitoneally (ip), 200 mg/kg of a solution (10 mg/ml) of phenol red; thirty minutes after the administration of phenol red, each animal, anaesthetised with tiletamine/zolazepam (telazol 30 mg/kg ip.) and xylazine (10 mg/kg ip), undergoes a bronchoalveolar lavage with 2 ml of saline solution (0.9% p/v). The lavage fluid collected is centrifuged at 1600 rpm for 10 min; NaOH (0.01 N) is added to the supernatants collected in a 2:1 ratio to permit the indicator to change colour from yellow to red. The absorbance of the various solutions is measured via a spectrophotometric reading at 546 nm and the concentration of phenol red (mg/ml) is derived therefrom using a standard curve (0.055 - 10 mg/ml) of phenol red as a reference.

### 2. Anti-inflammatory and antioxidant activity

The anti-inflammatory and antioxidant activity of the various formulations of *C.islandica* is evaluated *in vitro* in a line of murine macrophages (J774A.1) stimulated with bacterial endotoxin (LPS) according to a previously described procedure (lalenti et al., Mol Pharmacol 2005; 67:1620-1628).

### Murine macrophages J774

The cell line of murine macrophages J774 is cultured in an appropriate medium (Dulbecco's modified Eagles medium; DMEM) supplemented with glutamine 2 mM, Hepes 25 mM, penicillin (100 U/mL), streptomycin (100 µg/mL), 10% of fetal bovine serum (FBS) and 1.2% sodium pyruvate. The cells are seeded in Petri dishes with a diameter of 10 cm at a density of 1×10⁶ cells/dish. On the day of the experiment, the cells are seeded in 24-well plates at a density of 2.5×10⁵ cells/mL/well. After 18 hours the cells are pre-treated for 2 hours with the compounds to be tested (table 2) and stimulated with LPS of *Escherichia Coli* (Serotype 0111:B4) 10 µg/mL for 24 hours. Dexamethasone sodium phosphate (1µM) is used as the reference drug. At the end of the incubation the cell culture supernatant is collected; the nitrites and pro-inflammatory cytokines (e.g. TNFα) will be assayed in the latter.

### Cell viability

In preliminary experiments, the cytotoxicity of the substances to be tested (table 2) was evaluated by determination of cellular respiration. Cellular respiration, which is a measure of cell viability, is evaluated by reduction of 3-(4,5-dimethylthiazol-2-il)-2,5- diphenyltetrazolium bromide (MTT) to formazan (*Mosmann et al., 1983*), a reaction dependent on mitochondria. The cells are seeded in 96-well plates and treated as described above. At the end of the 24-hour period, the cells are incubated with MTT (0.2 mg/mL) for 1 hour. The culture medium is subsequently aspirated and the cells solubilised in DMSO (100 µL). The reduction of MTT to formazan in the cells is evaluated by measuring absorbance at 550 nm (OD₅₅₀).

### Nitrite dosage

The concentration of nitrites, a stable, measurable metabolite of nitrogen monoxide, is measured in the cell culture supernatant using the Griess colorimetric method, by adding 100 µL of Griess reagent (0.1% of naphthyl ethylenediamine hydrochloride in distilled H₂O and 1% *sulfanilamide* in H₃PO₄ at 5%; vol. 1:1) to 100 µL of the samples. After 10 minutes of incubation the absorbance of the chromophore is measured in a reader for microplates at a wavelength of 550 nm (OD₅₅₀). The nitrite concentration is determined by comparing the absorbance values read with those of a standard curve of sodium nitrite, prepared in the cell culture medium.

### Cytokine dosage

The concentration of the pro-inflammatory cytokines (e.g. TNFα) is measured in the cell culture supernatant by means of an immunoenzymatic assay.

### Measurement of the release of inflammatory mediators

The anti-inflammatory activity *in vitro* can also be analysed by measuring the secretion of IL-10 and IL-12p40 by human dendritic cells derived from monocytes[16].

### Measurement of the release of interleukin 8 (IL-8), interleukin 6 (IL-6) and RANTES.

Aquino *et al.* report the following method. Use is made of 1x10⁶ cells, which are allowed to adhere to the plate and are then treated for 2 hours with the extract whose activity it is desired to measure, and stimulated with TNF-α for 14 hours. The release of IL-8, IL-6 and RANTES is measured by means of the ELISA test and the cytokine concentration is expressed as pg/ml/10⁶ cells and as a percentage of the control in the absence of any stimulation or treatment[17].

### Test of lipoxygenase activity

Reaction medium: tris-HCI buffer. 100 mg of enzyme, linoleic acid solution. The inhibitory effect of the extract of interest was tested by adding the stock solution of the extract at different volumes, and measuring the absorbance at 234 nm. The activity of the enzyme is monitored as an increase in absorbance in relation to the formation of hydroperoxylinoleic acid[18].

The antioxidant activity can be evaluated by choosing one of the following proposed methods.

### DPPH

The antioxidant activity *in vitro* can be measured by means of the DPPH (2,2-diphenyl-1-picrylhydrazyl) test, which measures anti-free radical activity. Experimental procedure: preparation of stock solutions of the extract in 5% DMSO in a concentration of 1µg/ml. The solution was then diluted at various concentrations expressed in µg/ml. The diluted solutions were mixed with the 0.05 mg/ml methanolic DPPH solution. The mixtures were analysed at 517 nm, using methanol as the blank[11].

EC₅₀, i.e. the mean concentration with scavenging activity is measured using the Litchfield and Wilcoxon test as the concentration in µg/ml of sample necessary to reduce the initial concentration of DPPH by 50%[12].

### Reducing power

Experimental procedure: preparation of stock solutions of the extract in 5% DMSO in a concentration of 1µg/ml. The solution was then diluted at various concentrations expressed in µg/ml. 1 ml of each extract was mixed with phosphate buffer and potassium ferrocyanide and left at 50°C for 20 minutes; then trichloroacetic acid was added and the whole mixture centrifuged. The supernatant, mixed with distilled water and ferric chloride, was analysed at 700 nm. An increase in absorbance indicates an increase in the reducing power.

### Scavenging activity on superoxide anion

Experimental procedure: preparation of stock solutions of the extract in 5% DMSO in a concentration of 1µg/ml. The solution was then diluted at various concentrations expressed in µg/ml. 0.1 ml of each sample was made to react with NBT (nitroblue tetrazolium) and NADH; the reaction was started with PMS (phenazine methosulphate). The analysis was conducted by UV-Vis spectrophotometry at 560nm, considering a reduction in the absorbance as an indicator of the increased scavenging activity on the superoxide anion radical[11].

### Content of total phenolic compounds

The Folin-Ciocalteu method is used. The samples are placed in a test tube and the Folin-Ciocalteu reagent and sodium carbonate are added. The measurement is performed by spectrophotometry at 760 nm.

### Other methods used to measure antioxidant activity

### ABTS

This test measures anti-free radical activity using 2,2-azino-bis-(3-*ethylbenzthiazoline-6-sulphonic acid).* This compound can be generated by the enzymatic system made up of hydrogen peroxide and horseradish peroxidase. The ABTS* radical, a chromogen, is stable at room temperature, but not above 35°C and at a pH greater than 7.5. Furthermore, the ABTS/ABTS* ratio in the medium influences stability; it reacts with ascorbic acid in a constant manner with a stoichiometry of 1 mol of ascorbic acid per 2 mol of reduced ABTS*. The reaction is monitored via UV-Vis spectroscopy at 414 nm[13], by measuring the reduction in absorbance. The method provides for the addition of the sample after the formation of the radical in order to prevent interactions with the reagents.[14]

It enables a measurement of the antioxidant activity both of hydrophilic and lipophilic substances and is rapid.

### ORAC (oxygen radical absorption capacity assay)

This test is based on the production *in situ* of peroxyl free radicals generated with an azo compound, 2,2-azobis (2-methylpropionamidine) dihydrochloride (AAPH). AAPH generates free radicals, which, in the presence of oxygen, generate peroxyl radicals that interact with a fluorescent probe, changing its intensity and increasing the frequency of fluorescent decay. In the presence of antioxidants, fluorescence decay is inhibited. A calculation is made of the area beneath the fluorescent curve (micromoles of Trolox per ml). If the fluorescent probe is fluorescein (excitation at 490 nm and emission at 514nm), a pH at 7.40 is used[15]. Other probes can be beta-phycoerythrin and pyrogallol.

### 3. Antibacterial activity

The potential antibacterial effect of the various formulations of *C. Islandica* was tested on the main Gram-positive and Gram-negative bacteria responsible for respiratory disorders (*Staphylococcus aureus, Psudomonas aeruginosa. E. coli*) using the resazurin colorimetric assay according to a previously described procedure (Grujicic et al., Cytotechnology 2014; 66:803-813).

In order to evaluate the antibacterial and antimycotic activity of our formulation it is possible to use different methods described in the literature. The antibacterial and antimycotic activity is determined on the species of bacteria and fungi which most frequently affect the respiratory tract. The bacterial cultures are maintained on an agar gel, the fungal ones are maintained on dextrose.

The method envisages making microdilutions of the formulation [9]. In order to determine the MIC (minimum inhibitory concentration), it is possible to use the resazurin assay, described by Sarker et al. [10]. Resazurin is a redox indicator used in the evaluation of microbial growth. It has a non-fluorescent blue colour, but can be oxidised to resorufin, which has fluorescent pink colour, by the oxidoreductase present in the bacterial cells still alive. After the Petri plates have been prepared with the bacterial cultures, 10 µL of a resazurin solution is added and a solution of the formulation is added to each Petri plate at increasing concentrations [9]. After a period of incubation, the antibacterial activity is evaluated: the first plate that shows a blue colour (which indicates the absence of bacterial growth) is the one containing the minimum inhibitory concentration inhibiting bacterial growth. Three tests are carried out and the mean value of the MICs observed in the resofurin test is calculated to determine the MIC for each bacterial strain [10].

### REFERENCES

[1] R. Chakraborty, B. De, N. Devanna, and S. Sen, "Antitussive, expectorant activity of Marsilea minuta L., an Indian vegetable.," J. Adv. Pharm. Technol. Res., vol. 4, no. 1, pp. 61-4, Jan. 2013.
[2] K. J. Song, Y. J. Shin, K. R. Lee, E. J. Lee, Y. S. Suh, and K. S. Kim, "Expectorant and Antitussive Effect of Hedera helix and Rhizoma coptidis extracts mixture," Yonsei Med. J., vol. 56, no. 3, pp. 819-824, 2015.
[3] Y. Ge, F. Zhang, Q. Qin, Y. Shang, and D. Wan, "In Vivo Evaluation of the Antiasthmatic, Antitussive , and Expectorant Activities and Chemical Components of Three Elaeagnus Leaves," vol. 2015, 2015.
[4] T. Guo, J. Qing Wei, and J. Ping Ma, "Antitussive and expectorant activities of Potentilla anserina.," Pharm. Biol., vol. 54, no. 5, pp. 807-11, May 2016.
[5] K. Shin, T. Kim, J. Kyung, D. Kim, D. Park, and E. Choi, "Effectiveness of the combinational treatment of Laminaria japonica and Cistanche tubulosa extracts in hair growth," Lab Anim Res, vol. 31, no. 1, pp. 24-32, 2015.
[6] G. M. De Barros Alves, M. B. De Sousa Maia, E. De Souza Franco, A. M. Galv??o, T. G. from Silva, R. M. Gomes, M. B. Martins, E. P. from Silva Falc??o, C. M. M. B. De Castro, and N. H. from Silva, "Expectorant and antioxidant activities of purified fumarprotocetraric acid from Cladonia verticillaris lichen in mice," Pulm. Pharmacol. Ther., vol. 27, no. 2, pp. 139-143, 2014.
[7] R. Lizio, a Westhof, C. M. Lehr, and T. Klenner, "Oral endotracheal intubation of rats for intratracheal instillation and aerosol drug delivery.," Lab. Anim., vol. 35, no. 3, pp. 257-60, 2001.
[8] I. Kohler, R. Meier, A. Busato, G. Neiger-Aeschbacher, and U. Schatzmann, "Is carbon dioxide (CO2) a useful short acting anaesthetic for small laboratory animals?," Lab. Anim., vol. 33, no. 2, pp. 155-161, Apr. 1999.
[9] D. Grujičić, I. Stošić, M. Kosanic, T. Stanojković, B. Ranković, and O. Milošević- Djordjević, "Evaluation of in vitro antioxidant, antimicrobial, genotoxic and anticancer activities of lichen Cetraria islandica," Cytotechnology, pp. 1-11, 2014.
[10] S. D. Sarker, L. Nahar, and Y. Kumarasamy, "Microtitre plate-based antibacterial assay incorporating resazurin as an indicator of cell growth, and its application in the in vitro antibacterial screening of phytochemicals," Methods, vol. 42, no. 4, pp. 321-324, 2007.
[11] D. Grujič̌ić, I. Stošić, and M. Kosanić, "Evaluation of in vitro antioxidant, antimicrobial, genotoxic and anticancer activities of lichen Cetraria islandica," Cytotechnology, 2014.
[12] F. Sansone, T. Mencherini, and P. Picerno, "Microencapsulation by spray drying of Lannea microcarpa extract: technological characteristics and antioxidant activity," JPP Res, 2014.
[13] "An end-point method for estimation of the total antioxidant activity in plant material-00463537c5d0b00a36000000.pdf.".
[14] D. Villaño and M. Fernández-Pachón, "Comparison of antioxidant activity of wine phenolic compounds and metabolites in vitro," Anal. Chim...., 2005.
[15] S. Litescu and S. Eremia, "The Use of Oxygen Radical Absorbance Capacity (ORAC) and Trolox Equivalent Antioxidant Capacity (TEAC) Assays in the Assessment of Beverages'," ... Impact ...,2014.
[16] S. Omarsdottir, E. S. Olafsdottir, and J. Freysdottir, "Immunomodulating effects of lichen-derived polysaccharides on monocyte-derived dendritic cells.," Int. Immunopharmacol., vol. 6, no. 11, pp. 1642-1650, Nov. 2006.
[17] R. P. Aquino, A. Santoro, and L. Prota, "Composition and anti-inflammatory activity of extracts from three Paeonia species," ..., 2014.
[18] L. Rackova, M. Oblozinsky, D. Kostalova, V. Kettmann, and L. Bezakova, "Free radical scavenging activity and lipoxygenase inhibition of Mahonia aquifolium extract and isoquinoline alkaloids.," J. Inflamm. (Lond)., vol. 4, no. 1, p. 15, 2007.

## Claims

1. A pharmaceutical composition comprising:
- a mixture which comprises or, alternatively, consists of *Cetraria islandica Ach.,* sodium hyaluronate and a saline solution with an osmolarity such as to ensure that said composition has an osmolarity greater than or equal to 300 mOsm/l and, optionally,
- pharmaceutically acceptable excipients, additives and ingredients;
for use as a medicament in the treatment of respiratory tract disorders/pathologies selected from among rhinitis, sinusitis, asthma, pharyngitis (pharyngotonsillitis), epiglottitis, laryngitis, bronchiolitis, cystic fibrosis and bronchiectasis.

2. The composition for use according to claim 1, wherein the *Cetraria islandica Ach.* is present as a fluid, dry or glyceric extract; preferably, the *Cetraria islandica Ach.* is present in a concentration by weight comprised from 0.001% to 10%, preferably in a concentration by weight comprised from 0.1% to 5%, and even more preferably in a concentration by weight comprised from 0.1% to 2.5%, relative to the total weight of the composition.

3. The composition for use according to claim 1 or 2, wherein the sodium hyaluronate is present in a concentration by weight comprised from 0.01% to 5%, preferably in a concentration by weight comprised from 0.05% to 2.5%, or from 0.3% to 2.5%, and even more preferably in a concentration by weight comprised from 0.1% to 1% or from 0.5% to 1%, relative to the finished product/final composition.

4. The composition for use according to any one of claims 1-3, wherein the average molecular weight of the sodium hyaluronate used together with the *Cetraria islandica Ach.* is comprised from 100 kDa to 3000 kDa; preferably, the average molecular weight of the sodium hyaluronate is comprised from 200 kDa to 1500 kDa; even more preferably, the average molecular weight of the sodium hyaluronate is comprised from 300 kDa to 1000 kDa.

5. The composition for use according to any one of claims 1-4, wherein the saline solution is based on an osmotically active salt, preferably sodium chloride, in an amount such as to ensure that said composition has an osmolarity greater than or equal to 300 mOsm/l, preferably comprised from greater than 300 mOsm/l to 10000 mOsm/l or from greater than 300 mOsm/l to 2000 mOsm/l; even more preferably, it has an osmolarity comprised from greater than 300 mOsm/l to 3000 mOsm/l or from 400 mOsm/l to 1000 mOsm/l, for example 360, 500, 600, 700, 800, 900, 1000, 1220, 1660, 2100, 2380 or 2900 mOsm/l.

6. The composition for use according to any one of claims 1-5, wherein said composition is for use in the treatment of adolescents and subjects of paediatric age.

7. The composition for use according to any one of claims 1-6, wherein said composition is for use in the treatment of the respiratory tract pathologies associated with mucus hypersecretion, preferably in adolescents and subjects of paediatric age.

8. The composition for use according to any one of claims 1-7, wherein said composition is for oral, nasal or inhalatory use, by spray or aerosol application/administration.

9. The composition for use according to any one of claims 1-8, wherein said composition is administered by nebulization with a special device, such as a pneumatic or ultrasonic nebulizer, or by using a spray, or by direct nasal instillation.

## Patentansprüche

1. Pharmazeutische Zusammensetzung, umfassend:
- eine Mischung umfassend bzw. - alternativ - bestehend aus *Cetraria islandica Ach.,* Natriumhyaluronat und einer Salzlösung mit einer Osmolarität, um zu gewährleisten, dass die Zusammensetzung eine Osmolarität größer oder gleich 300 mOsm/L aufweist, und, gegebenenfalls,
- pharmazeutisch akzeptable Hilfsstoffe, Zusatzstoffe und Inhaltsstoffe,
zur Verwendung als Medikament bei der Behandlung von Atemwegsstörungen/-pathologien ausgewählt aus Rhinitis, Sinusitis, Asthma, Pharyngitis (Pharyngotonsillitis), Epiglottiditis, Laryngitis, Bronchiolitis, Mukoviszidose und Bronchiektase.

2. Zusammensetzung nach Anspruch 1, wobei *Cetraria islandica Ach.* als Flüssig-, Trocken- oder Glycerinextrakt vorliegt; vorzugsweise liegt *Cetraria islandica Ach.* in einer Gewichtskonzentration von 0.001 % bis 10 %, vorzugweise 0,1 % bis 5 %, und noch bevorzugter 0,1 % bis 2,5 %, bezogen auf die Gesamtkonzentration der Zusammensetzung, vor.

3. Zusammensetzung zur Verwendung nach Anspruch 1 oder 2, wobei das Natriumhyaluronat in einer Gewichtskonzentration von 0,01 % bis 5 %, vorzugsweise 0,05 % bis 2,5 % oder 0,3 % bis 2,5 %, und noch bevorzugter 0,1 % bis 1 % oder 0,5 % bis 1 %, bezogen auf das Fertigprodukt/die Endzusammensetzung, vorliegt.

4. Zusammensetzung zur Verwendung nach einem der Ansprüche 1 bis 3, wobei das durchschnittliche Molekulargewicht des zusammen mit *Cetraria islandica Ach.* verwendeten Natriumhyaluronats 100 kDa bis 3000 kDa, vorzugsweise 200 kDa bis 1500 kDa, und noch bevorzugter 300 kDa bis 1000 kDa, umfasst.

5. Zusammensetzung zur Verwendung nach einem der Ansprüche 1 bis 4, wobei die Salzlösung auf einem osmotisch aktiven Salz, vorzugweise Natriumchlorid, in einer Menge beruht, die gewährleistet, dass die Zusammensetzung eine Osmolarität von 300 mOsm/L oder mehr, vorzugsweise mehr als 300 mOsm/L bis 10000 mOsm/L, oder von mehr als 300 mOsm/L bis 2000 mOsm/L; noch bevorzugter von mehr als 300 mOsm/L bis 3000 mOsm/L oder von 400 mOsm/L bis 1000 mOsm/L, zum Beispiel 360, 500, 600, 700, 800, 900, 1000, 1220, 1660, 2100, 2380 oder 2900 mOsm/L, aufweist.

6. Zusammensetzung zur Verwendung nach einem der Ansprüche 1 bis 5, wobei die Zusammensetzung zur Behandlung von Jugendlichen und pädiatrischen Patienten verwendet wird.

7. Zusammensetzung zur Verwendung nach einem der Ansprüche 1 bis 6, wobei die Zusammensetzung bei der Behandlung von mit einer übermäßigen Absonderung von Mucus in Verbindung gebrachten Atemwegspathologien, vorzugweise bei Jugendlichen und pädiatrischen Patienten, verwendet wird.

8. Zusammensetzung zur Verwendung nach einem der Ansprüche 1 bis 7, wobei die Zusammensetzung zur oralen, nasalen oder inhalativen Verwendung bei Anwendung/Verabreichung durch Spray oder Aerosol dient.

9. Zusammensetzung zur Verwendung nach einem der Ansprüche 1 bis 8, wobei die Zusammensetzung verabreicht wird durch Vernebeln mithilfe einer besonderen Vorrichtung, wie einem pneumatischen Vernebler oder Ultraschallvernebler, oder durch Verwendung eines Sprays oder durch direkte Einträufelung durch die Nase.

## Revendications

1. Composition pharmaceutique comprenant :
- un mélange qui comprend ou, en variante, consiste en, *Cetraria islandica Ach.,* du hyaluronate de sodium et une solution salée ayant une osmolarité telle qu'il soit assuré que ladite composition ait une osmolarité supérieure ou égale à 300 mOsm/l, et éventuellement
- des excipients, additifs et ingrédients pharmaceutiquement acceptables ;
pour une utilisation en tant que médicament dans le traitement des troubles/pathologies des voies respiratoires choisis parmi la rhinite, la sinusite, l'asthme, la pharyngite (pharyngo-amygdalite), l'épiglottite, la laryngite, la bronchiolite, la mucoviscidose et la bronchiectasie.

2. Composition selon la revendication 1, dans laquelle *Cetraria islandica Ach.* est présent sous la forme d'un extrait fluide, sec ou glycérique ; de préférence *Cetraria islandica Ach.* est présent à une concentration en poids comprise entre 0,001 % et 10 %, de préférence à une concentration en poids comprise entre 0,1 % et 5 %, et mieux encore à une concentration en poids comprise entre 0,1 % et 2,5 %, par rapport au poids total de la composition.

3. Composition pour une utilisation selon la revendication 1 ou 2, dans laquelle le hyaluronate de sodium est présent à une concentration en poids comprise entre 0,01 % et 5 %, de préférence à une concentration en poids comprise entre 0,05 % et 2,5 %, ou entre 0,3 % et 2,5 %, et mieux encore à une concentration en poids comprise entre 0,1 % et 1 % ou entre 0,5 % et 1 %, par rapport au produit fini/à la composition finale.

4. Composition pour une utilisation selon l'une quelconque des revendications 1 à 3, dans laquelle la masse moléculaire moyenne du hyaluronate de sodium utilisé conjointement avec *Cetraria islandica Ach.* est comprise entre 100 kDa et 3 000 kDa ; de préférence la masse moléculaire moyenne du hyaluronate de sodium est comprise entre 200 kDa et 1 500 kDa ; mieux encore la masse moléculaire moyenne du hyaluronate de sodium est comprise entre 300 kDa et 1 000 kDa.

5. Composition pour une utilisation selon l'une quelconque des revendications 1 à 4, dans laquelle la solution salée est à base d'un sel ayant une activité osmotique, de préférence le chlorure de sodium, en une quantité telle qu'il soit assuré que ladite composition ait une osmolarité supérieure ou égale à 300 mOsm/l, de préférence comprise entre plus de 300 mOsm/l et 10 000 mOsm/l ou entre plus de 300 mOsm/l et 2 000 mOsm/l ; mieux encore elle a une osmolarité comprise entre plus de 300 mOsm/l et 3 000 mOsm/l ou entre 400 mOsm/l et 1 000 mOsm/l, par exemple 360, 500, 600, 700, 800, 900, 1 000, 1 220, 1 660, 2 100, 2 380 ou 2 900 mOsm/l.

6. Composition pour une utilisation selon l'une quelconque des revendications 1 à 5, laquelle composition est destinée à être utilisée dans le traitement d'adolescents et de sujets pédiatriques.

7. Composition pour une utilisation selon l'une quelconque des revendications 1 à 6, laquelle composition est destinée à être utilisée dans le traitement des pathologies des voies respiratoires associées à une hypersécrétion de mucus, de préférence chez les adolescents et les sujets pédiatriques.

8. Composition pour une utilisation selon l'une quelconque des revendications 1 à 7, laquelle composition est destinée à être utilisée par voie orale ou nasale ou par inhalation, par application/administration d'un spray ou d'un aérosol.

9. Composition pour une utilisation selon l'une quelconque des revendications 1 à 8, laquelle composition est administrée par nébulisation avec un dispositif spécial, tel qu'un nébuliseur pneumatique ou ultrasonique, ou par utilisation d'un spray, ou par instillation nasale directe.
